(19) Europäisches Patentamt
European Patent Office
Office européen des brevets

(11) Veröffentlichungsnummer: **0 464 471 A2**

# (12) EUROPÄISCHE PATENTANMELDUNG

(21) Anmeldenummer: **91110055.0**

(22) Anmeldetag: **19.06.91**

(51) Int. Cl.⁵: **C07C 63/72**, C07C 51/265

(30) Priorität: **25.06.90 DE 4020185**

(43) Veröffentlichungstag der Anmeldung: **08.01.92 Patentblatt 92/02**

(84) Benannte Vertragsstaaten: **CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **Röhrscheid, Freimund**
**Amselweg 24**
**W-6233 Kelkheim(DE)**
Erfinder: **Appel, Wolfgang**
**Taunusstrasse 74A**
**W-6233 Kelkheim (Taunus)(DE)**
Erfinder: **Siegemund, Günter**
**Frankfurter Strasse 21**
**W-6238 Hofheim(DE)**

## (54) Teilfluorierte Dicarbonsäure sowie deren Säurechlorid, Verfahren zu ihrer Herstellung und ihre Verwendung.

(57) Verbindung der Formel (I)

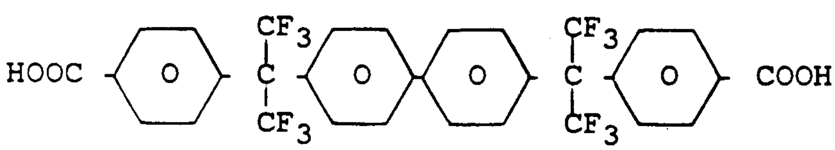

(I)

sowie deren Säurechlorid werden hergestellt durch Luftoxidation in Gegenwart eines Katalysatorgemisches aus den Ionen von Kobalt, Mangan und Brom in einem sauren organischen Medium. Die Verbindungen können zur Herstellung von linearen Polycarbonsäureamiden und -carbonsäureestern verwendet werden.

EP 0 464 471 A2

Die Erfindung betrifft eine teilfluorierte Dicarbonsäure, im speziellen das 4,4'-Bis-[2-(4-carboxyphenyl)-hexafluorisopropyl]biphenyl, dessen Säurechlorid, Verfahren zu ihrer Herstellung und ihre Verwendung.

Aromatische Dicarbonsäurehalogenide, die gemäß einer allgemeinen Formel mit mehreren Variablen auch teilfluorierte Biphenyle enthalten können, sind als Reaktionskomponente zur Darstellung von Polyketonen in Gegenwart von Fluoralkansulfonsäuren bekannt [EP-A-0063874]. Ein Verfahren zur Herstellung der Dicarbonsäurehalogenide wird nicht offenbart. Darüber hinaus werden in den Beispielen keine teilfluorierten oder biphenylgruppenhaltigen Dicarbonsäurehalogenide beschrieben.

Weiterhin sind teilfluorierte Dicarbonsäuren mit einer Diphenyletherbrücke bekannt, die durch Luftoxidation in saurem Medium bei erhöhtem Druck und erhöhter Temperatur in Gegenwart eines Katalysatorgemisches hergestellt wurden [DE-A-37 39 797].

Gegenstand der Erfindung ist eine Verbindung der Formel

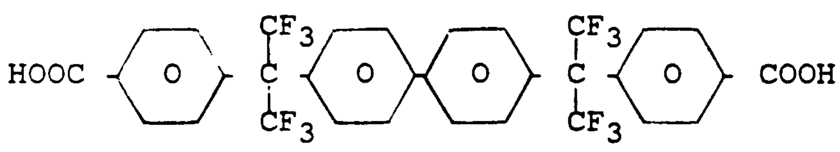

(I)

sowie deren Säurechlorid, Verfahren zu ihrer Herstellung und ihre Verwendung als Monomerkomponente für die Herstellung von Polykondensaten wie Polyestern oder Polyamiden.

Die Herstellung der Verbindung gemäß der Erfindung erfolgt durch Oxidation des 4,4'-Bis[2-(4-alkylphenyl)hexafluorisopropyl]biphenyls mit molekularem Sauerstoff in einem sauren organischen Medium, wobei das saure Medium zu mindestens 40 Gew.-% aus einer Monocarbonsäure mit 1 bis 4 Kohlenstoffatomen, insbesondere Essigsäure oder Propionsäure oder deren Mischungen, besteht, in Gegenwart einer Katalysatorkombination aus den Ionen von Kobalt, Mangan und Brom. Daneben können noch Cerionen vorhanden sein. Essigsäure ist wegen ihrer größeren Beständigkeit gegen einen oxidativen Abbau vorzuziehen. Das Verhältnis von saurem Medium zu der zu oxidierenden Biphenylverbindung kann maximal 40:60 Gew.-%, bezogen auf die Gesamtreaktionsmasse, betragen.

Die eingesetzte Biphenylverbindung

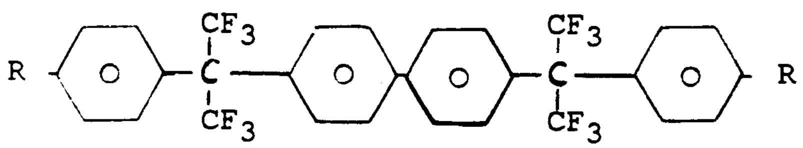

(II),

in der R gleich niederes Alkyl mit 1-4 C-Atomen ist, wobei Alkyl vorzugsweise Methyl, Ethyl und Isopropyl, insbesondere Methyl ist, wird im allgemeinen nach drei verschiedenen Methoden hergestellt, und zwar:

a) durch Kondensation von einem Mol eines Dicarbinols der Formel

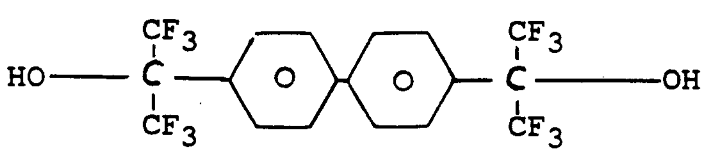

(III)

mit mindestens 2 Mol einer Verbindung mit der Formel

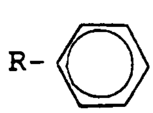

(IV),

wobei R die vorgenannte Bedeutung hat,

oder

b) durch Kondensation von mindestens 2 Mol einer Verbindung der Formel

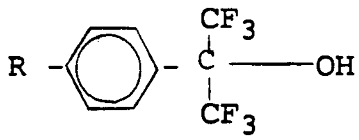

(V),

wobei R die vorgenannte Bedeutung hat,
mit einem Mol Biphenyl (VI), jeweils in Gegenwart von Fluorwasserstoff

oder

c) durch Bildung der Kohlenstoff-Kohlenstoff-Bindung zwischen 2 gleichen teilfluorierten aromatischen Verbindungen der Formel

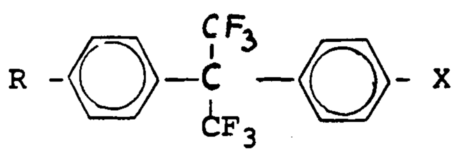

(VII),

wobei R die vorgenannte Bedeutung hat,
nach einem literaturbekannten Verfahren, das sich zur Bildung von Aryl-Arylbindungen eignet, z.B. J. Org. Chem. 51, 2627 (1986). X ist Halogen, vorzugsweise Chlor.

Verbindungen der Formel (III), die beim Vorgehen nach a) eingesetzt werden, sind in der US-Patentschrift 3,355,500 und in J. Org. Chem. 30, 998 (1965) beschrieben. Verbindungen der Formel (V), die nach Methode b) zu den Verbindungen gemäß Formel (II) umgesetzt werden, sind ebenfalls in J. Org. Chem. 30, 998-1001 (1965) beschrieben.

Die Reaktion nach Methode a) und b) wird bei einer Temperatur von 80 bis 180 °C, vorzugsweise von 100 bis 160 °C, durchgeführt.

Für die Reaktion nach Methode a) und b) ist ein Zeitraum von 20 bis 90 Stunden, vorzugsweise von 40 bis 70 Stunden, erforderlich.

Das Molverhältnis der eingesetzten Reaktionspartner wird bestimmt bei Methode a) durch das Verhältnis der Verbindung (III) zu Verbindung (IV), sowie bei Methode b) durch das Verhältnis des Biphenyls zu Verbindung (V); es beträgt jeweils mindestens 1:2, vorzugsweise 1:2,2 bis 1:4,4.

Der Anteil an Fluorwasserstoff, der bei der Reaktion zur Herstellung der Verbindungen gemäß Formel (II) notwendig ist, wird bei Methode a) auf die Verbindung (III) bezogen und liegt im allgemeinen bei einem Molverhältnis von 1:7 bis 1:25, vorzugsweise 1:8 bis 1:12. Bei der Methode b) beträgt das Molverhältnis der Verbindungen (V) zu Fluorwasserstoff im allgemeinen 1:6 bis 1:15, vorzugsweise 1:8 bis 1:12.

Zur Aufarbeitung des Reaktionsgutes wird im allgemeinen der Fluorwasserstoff nach Beendigung der Reaktion bei ca. 80 °C aus dem Reaktor abgegast und der verbleibende Rückstand, gegebenenfalls nach Verdünnen mit einem organischen Lösungsmittel, dem Reaktor entnommen, vorzugsweise bei einer Temperatur von 20 bis 30 °C.

Als Lösungsmittel, die dabei verwendet werden können, eignen sich aliphatische Kohlenwasserstoffe mit 5 bis 10 C-Atomen, aromatische Kohlenwasserstoffe mit 6 bis 8 C-Atomen und ein- oder mehrfach chlorierte aliphatische Kohlenwasserstoffe mit 1 bis 4 C-Atomen im Alkylrest. Beispiele hierfür sind n-Hexan, n-Heptan, Toluol, die verschiedenen Xylole, sowie Di- und Trichlormethan, vorzugsweise Toluol, Di- oder Trichlormethan.

Das erhaltene Rohgemisch wird mit Wasser vermischt, gewaschen und abgetrennt. Im allgemeinen fallen die gereinigten Produkte als farblose Kristallisate an.

Zur weiteren Reinigung kann das Reaktionsgut einer Umkristallisation aus einem organischen Lösungsmittel unterworfen werden, oder es erfolgt ein Ausrühren in organischen Lösungsmitteln, vorzugsweise in Isopropanol, Methanol oder 1-Chlorpropan.

Die Herstellung der Verbindungen gemäß Formel (VII) ist nach bekannten Verfahren möglich aus Verbindungen der Formel (V) und Arylhalogeniden.

Die Bildung der Aryl-Arylbindung zwischen zwei Komponenten nach Formel (VII) wird durchgeführt in einem polaren aprotischen Lösungsmittel wie Dimethylacetamid oder Dimethylformamid in Gegenwart eines Gemisches aus 1 bis 10 Mol-%, vorzugsweise 3 bis 6 Mol-% eines Nickel-(II)-Salzes, vorzugsweise $NiCl_2$ oder $NiBr_2$, und 5 bis 40 Mol-%, vorzugsweise 20 bis 30 Mol-%, einer organischen Phosphor-(III)-Verbindung, vorzugsweise Triphenylphosphin, und Zinkpulver in einer Menge von 120 bis 160 Mol-%, bezogen auf das eingesetzte Arylhalogenid.

Die Reaktion wird in einer Inertgasatmosphäre, insbesondere von Stickstoff oder Argon, bei einer Temperatur von 40 bis 80 °C ausgeführt; die Reaktion dauert 2 bis 8 Stunden.

Der Feststoffanteil wird abfiltriert und das Filtrat nach Zusatz eines nicht mit Wasser mischbaren Lösungsmittels, z.B. ein ein- oder mehrfach chlorierter aliphatischer Kohlenwasserstoffe mit 1-4 Kohlenstoffatomen im Alkylrest, insbesondere Di- oder Trichlormethan, Essigsäureester oder Diethylether, mehrfach mit Wasser gewaschen. Dabei stellt sich eine Phasentrennung ein. Nach Trocknung der organischen Phase wird das Lösungsmittel abdestilliert und das zurückbleibende Produkt durch Umkristallisation gereinigt.

Für den vollständigen Ablauf der Oxidation sind Bromidionen unbedingt notwendig. Die beiden Schwermetallsalze, insbesondere die des Kobalts und Mangans, werden im allgemeinen im Verhältnis von 3:1 bis 1:3, vorzugsweise 1:1 eingesetzt. Die Summe der Konzentrationen der beiden Kationen beträgt im allgemeinen 0,01 bis 0,2, vorzugsweise 0,02 bis 0,12 und insbesondere 0,04 bis 0,08 Grammatom/kg Gesamtmasse. Das Verhältnis der Summe der Metallsalze, in diesem Falle der von Kobalt und Mangan, zu Brom ist im allgemeinen 1:0,01 bis 1:2, vorzugsweise 1:0,025 bis 1:1 und insbesondere 1:0,05 bis 1:0,2.

Es ist auch möglich, zusätzlich zu den beiden Metallionen des Katalysators auch Cerionen einzusetzen. Diese katalysieren die Oxidation unvollständig oxidierter Zwischenstufen. Ihre Anwesenheit erhöht die Reinheit und die Ausbeute der teilfluorierten Dicarbonsäure. Die Cerionen werden dem Katalysator im Verhältnis der Summe der Kobalt- und Manganionen zu Cerionen wie 1:0,02 bis 1:2, vorzugsweise 1:0,05 bis 1:1 und insbesondere 1:0,2 bis 1:0,6, zugefügt. Vorzugsweise werden als Metallsalze die entsprechenden Acetate eingesetzt.

Brom kann in Form von Bromiden z.B. der Bromide der Alkalimetalle einschließlich des Ammoniumbromids und der Metalle Kobalt, Mangan und Cer, oder als Lösung von

Bromwasserstoff in Wasser oder Eisessig eingesetzt werden. Es können auch bromhaltige organische Verbindungen verwendet werden, die während der Oxidation zerfallen und Bromionen freisetzen, z.B. Tetrabrommethan.

Die Oxidation wird im allgemeinen bei einer Temperatur von 120 bis 220, vorzugsweise 140 bis 190 und insbesondere 155 bis 180 °C durchgeführt. Der Druck im Reaktor beträgt im allgemeinen 5 bis 40, vorzugsweise 10 bis 30 und insbesondere 14 bis 20 bar.

Für die erfindungsgemäße Verfahrensweise ist es günstig, daß die zur Oxidation benötigte Luft nahe dem Boden des Reaktors in die Flüssigphase eingeleitet und vorzugsweise durch starkes Rühren oder durch spezielle Düsen in der Flüssigphase fein verteilt wird. Besonders günstig ist es, ein Oxidationsgemisch einzuleiten, dessen Sauerstoffgehalt durch Beimischen von reinem Sauerstoff auf einen Anteil von über 21 Volumen-% erhöht wurde. Durch diese Maßnahme werden hohe Sauerstoff-Partialdrucke in den in die Flüssigphase eintretenden Gasblasen erreicht. Es ist vorteilhaft, wenn der SauerstoffPartialdruck an der Austrittsstelle der Einleitungsvorrichtung mindestens 1 bar, vorzugsweise 2 bis 15 und insbesondere 3 bis 10 bar, beträgt.

Es ist für die Verfahrensführung weiterhin günstig, daß der Restsauerstoffgehalt des Abgases bestimmte Werte nicht unterschreitet. Der Sauerstoff-Partialdruck wird definiert durch die Formel

$$P_{O_2} = \text{Volumen-\% } O_2 \cdot (P_{ges} - P_{ac})$$

d.h. er ist das mathematische Produkt aus dem Restsauerstoffgehalt und der Differenz aus dem Gesamtdruck und dem Essigsäuredampfdruck bei der vorliegenden Reaktionstemperatur. Dieser Sauerstoff-Partialdruck in der Gasphase über der Reaktionslösung soll 0,2 bar nicht unterschreiten und liegt bevorzugt bei 0,35 bis 2,8, insbesondere bei 0,45 bis 1,3 bar.

Zur Überführung in das Säurechlorid wird die nach dem erfindungsgemäßen Verfahren erhaltene Dicarbonsäure in bekannter Weise mit Thionylchlorid behandelt und nach bekannten Methoden aus der Reaktionslösung gewonnen.

Die erfindungsgemäßen Verbindungen werden vor allem zur Herstellung von linearen Polycarbonsäureamiden und -carbonsäureestern verwendet, die als Formkörper, Filme und Fasern hohe thermische

Stabilität, ausgezeichnete mechanische Eigenschaften, gute Transparenz, gute schmutzabweisende Eigenschaften und Strahlenbeständigkeit aufweisen.

**Beispiele**

**1) 4,4'-Bis[2-(4-carboxypbenyl)hexafluorisopropyl]biphenyl**

158,6 g 4,4'-Bis[2-(4-methylphenyl)hexafluorisopropyl]biphenyl, 4,96 g $Co(OAc)_2 \cdot 4H_2O$, 2,45 g $Mn(OAc)_2 \cdot 4H_2O$, 2,4 g HBr = 24 g einer 10 %igen HBr-Lösung in Eisessig und 500 g Eisessig wurden in einem Ein-Liter-Glasautoklaven, der mit Rührer, Gaseinleitrohr, Thermometer, Rückflußkühler und einem Sauerstoffmeßgerät in der Abgasleitung ausgerüstet war, vorgelegt.

Unter 16 bar Stickstoff wurde das Reaktionsgemisch auf 150°C erhitzt. Nach dem Einleiten von Luft durch das nah am Boden befindliche Einleitungsrohr setzte sofort die exotherme Reaktion unter Sauerstoffaufnahme ein, wobei die Temperatur bis auf 170-175°C stieg. Es wurde soviel Luft eingeleitet, daß der Sauerstoffrestgehalt im Abgas zwischen 5 und 9 Vol-% lag.

Die exotherme Reaktion dauerte 20 Minuten. Anschließend wurde die Zufuhr von Luft durch eine Mischung von Stickstoff-Sauerstoff (9:1) ersetzt und die Temperatur durch Heizen noch 40 Minuten bei 165°C gehalten.

Die abgekühlte (ca. 115°C) Reaktionslösung wurde in einen Zwei-Liter-Kolben gesaugt und unter Rühren auf 20°C abgekühlt. Die entstandene Kristallsuspension wurde abgesaugt. Der Filterkuchen wurde fünfmal mit jeweils 50 ml Eisessig gewaschen. Das feuchte Produkt wurde bei 70°C/65 mbar im leichten Luftstrom getrocknet.

Ausbeute : 159,9 g (92,1 % d.Th.)
Festpunkt: 292-294°C

Durch Einengen der Mutterlauge auf 70 g fielen weitere 7,3 g Dicarbonsäure (4,2 % d.Th.) aus.
Fp.: 285-289°C.

| Analyse für $C_{32}H_{18}F_{12}O_4$: | | | |
|---|---|---|---|
| ber.: | C 55,34 % | H 2,61 % | F 32,83 % |
| gef.: | C 55,4 % | H 2,7 % | F 32,6 % |

**2) 4,4'-Bis[2-(4-chlorcarbonylpbenyl)hexafluorisopropyl]biphenyl**

Zu einer Aufschlämmung von 4,4'-Bis[2-(4-carboxyphenyl)hexafluorisopropyl]biphenyl in Thionylchlorid wurden einige Tropfen Dimethylformamid hinzugegeben und die Mischung unter Rückflußbedingungen erhitzt, bis die Gasentwicklung beendet war. Der Überschuß an Thionylchlorid wurde abgezogen und Toluol zugegeben, um das restliche Thionylchlorid durch Destillation abzutrennen. Nachdem das Toluol entfernt worden war, wurde das Rohprodukt aus n-Hexan umkristallisiert.
Fp.: 154-158°C

**Patentansprüche**

1. Verbindung der Formel

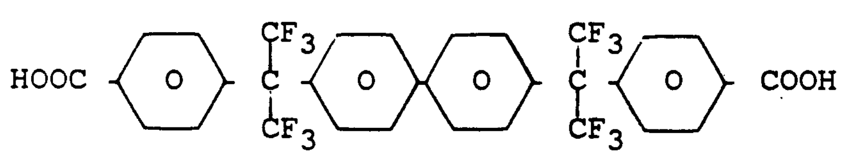

(I)

sowie deren Säurechlorid.

2. Verfahren zur Herstellung einer Verbindung der Formel

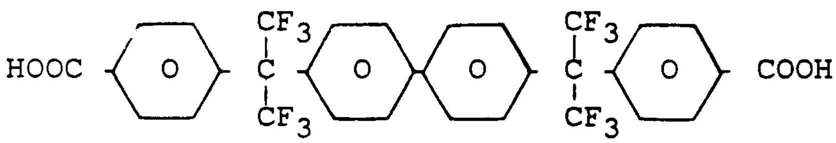

(I)

oder deren Säurechlorid durch Luftoxidation in saurem Medium bei erhöhtem Druck und erhöhter Temperatur in Gegenwart eines Katalysatorgemisches, dadurch gekennzeichnet, daß 4,4'-Bis[2-(4-methylphenyl)hexafluorisopropyl]biphenyl durch Einleiten von Luftsauerstoff in ein saures organisches Medium, welches aus einer Monocarbonsäure mit 1 bis 4 Kohlenstoffatomen besteht, bei einer Temperatur von 120 bis 220°C und einem Druck von 5 bis 40 bar in Gegenwart eines Gemisches von Verbindungen der Metalle Co und Mn sowie von Bromidionen oxidiert und als Produkt nach Formel (I) isoliert wird oder dieses unter Verwendung von Thionylchlorid in das Säurechlorid der Verbindung der Formel (I) überführt wird.

3. Verfahren nach Anspruch 2, dadurch gekennzeichnet, daß das saure organische Medium zu mindestens 40 Gew.-% aus Essigsäure und/oder Propionsäure besteht, bezogen auf das Gesamtgewicht.

4. Verfahren nach Anspruch 2 oder 3, dadurch gekennzeichnet, daß die Reaktionstemperatur 140 bis 190, insbesondere 155 bis 180°C beträgt.

5. Verfahren nach einem oder mehreren der Ansprüche 2 bis 4, dadurch gekennzeichnet, daß die Oxidation bei einem Druck von 10 bis 30, insbesondere von 14 bis 20 bar durchgeführt wird.

6. Verfahren nach einem oder mehreren der Ansprüche 2 bis 5, dadurch gekennzeichnet, daß der zur Oxidation eingesetzte Luftsauerstoff einen Sauerstoffgehalt von über 21 Vol-% besitzt und daß an der Einleitstelle des Sauerstoffs der Sauerstoffpartialdruck mindestens 1 bar, vorzugsweise 2 bis 15, insbesondere 3 bis 10 bar beträgt.

7. Verfahren nach einem oder mehreren der Ansprüche 2 bis 6, dadurch gekennzeichnet, daß der Sauerstoffpartialdruck in der Gasphase über dem Reaktionsmedium mindestens 0,2 bar, vorzugsweise 0,35 bis 2,8, insbesondere 0,45 bis 1,3 bar beträgt.

8. Verfahren nach einem oder mehreren der Ansprüche 2 bis 7, dadurch gekennzeichnet, daß das Verhältnis von Kobalt zu Mangan gleich 3:1 bis 1:3, vorzugsweise 1:1 ist, wobei die Summe der Konzentrationen der beiden Elemente Kobalt und Mangan gleich 0,01 bis 0,20 g-Atom/kg Gesamtreaktionsmasse, vorzugsweise 0,02 bis 0,12, insbesondere 0,04 bis 0,08 g-Atom/kg beträgt.

9. Verfahren nach einem oder mehreren der Ansprüche 2 bis 8, dadurch gekennzeichnet, daß das Verhältnis der Summe von Kobalt und Mangan zu Brom 1:0,01 bis 1:2, vorzugsweise 1:0,025 bis 1:1, insbesondere 1:0,05 bis 1:0,2 beträgt.

10. Verfahren nach einem oder mehreren der Ansprüche 2 bis 9, dadurch gekennzeichnet, daß als zusätzliches Metallion Cer im Katalysator enthalten ist, wobei das Molverhältnis der Summe von Kobalt und Mangan zu Cer 1:0,02 bis 1:2, vorzugsweise 1:0,05 bis 1:1, insbesondere 1:0,2 bis 1:0,6 beträgt.

11. Verfahren nach einem oder mehreren der Ansprüche 2 bis 10, dadurch gekennzeichnet, daß als Metallsalze die entsprechenden Acetate verwendet werden.

12. Verfahren nach einem oder mehreren der Ansprüche 2 bis 11, dadurch gekennzeichnet, daß Brom in Form von Bromiden oder als Lösung von Bromwasserstoff in Wasser oder Eisessig eingesetzt wird.

13. Verwendung der Verbindungen gemäß Anspruch 1 zur Herstellung von linearen Polycarbonsäureamiden und -carbonsäureestern.

14. Verwendung nach Anspruch 13, dadurch gekennzeichnet, daß Formkörper, Filme und Fasern hergestellt werden.